# EUROPEAN PATENT APPLICATION

(11) **EP 4 252 806 A1**
(43) Date of publication of application: **04.10.2023**
(21) Application number: 23164056.6
(22) Date of filing: 24.03.2023
(51) Int. Cl.: A61M 15/00, A61M 15/06, A61M 15/02, A61M 16/00

(54) **FLUID JET EJECTION DEVICE AND METHOD OF JETTING A FLUID THAT IS SYNCHRONIZED WITH BREATHING**

(30) Priority: 29.03.2022 US 202217706702
(71) Applicant: Funai Electric Co., Ltd., Daito, Osaka, 574-0013 (JP)
(72) Inventor: Marra III, Michael A., Lexington, KY, 40508 (US)
(74) Representative: Kurig, Thomas

(57) **Abstract**

A fluid jet ejection device and a method for controlling the delivering of a fluid to a user. The fluid jet ejection device includes a cartridge body; a fluid outlet nozzle attached to the cartridge body; a fluid jet ejection cartridge disposed in the cartridge body, the fluid jet ejection cartridge containing a fluid and an ejection head attached to the fluid jet ejection cartridge; wherein the ejection head contains a plurality of fluid ejectors thereon and a nozzle plate having a plurality of fluid ejection nozzles therein associated with the plurality of fluid ejectors configured to deliver the fluid to a user at a predetermined rate. A control system is disposed in the fluid jet ejection device that includes a differential pressure sensor for sensing an inhalation differential pressure. The control system is configured to terminate fluid delivery below a threshold inhalation differential pressure.

## Description

### TECHNICAL FIELD

The disclosure is directed to fluid jet ejection devices for delivering fluids to users and, in particular, to method for controlling the delivery of the fluids from the fluid jet ejection devices.

### BACKGROUND AND SUMMARY

Fluid jet ejection devices have been designed and used to eject ink onto a substrate. However, new uses for fluid jet ejection devices continue to evolve. For example, fluid jet ejection devices may be used for vapor or mist producing devices for drug delivery, such as nasal, oral, pulmonary, ophthalmic, and wound care application, and for ejecting a variety of non-aqueous fluids such as lubricants and fragrances.

Conventional nasal spray devices are designed to provide a mist of fluid that is injected into the nasal cavity. Nasal spray devices have become important methods for delivering therapeutic drugs to users. Therapeutic drugs are those drugs that are administered to treat or cure a user or cure a disease, or otherwise improve the health of a user. Such devices are more convenient to use than the administration of drugs through intravenous (IV) or injection. Spray devices also provide higher bioavailability of drugs compared to oral administration of drugs. The absorption of drugs through spray devices is more rapid compared to the absorption of drugs administered orally since drugs delivered by nasal spray devices directly enter the blood stream making their effect more immediate.

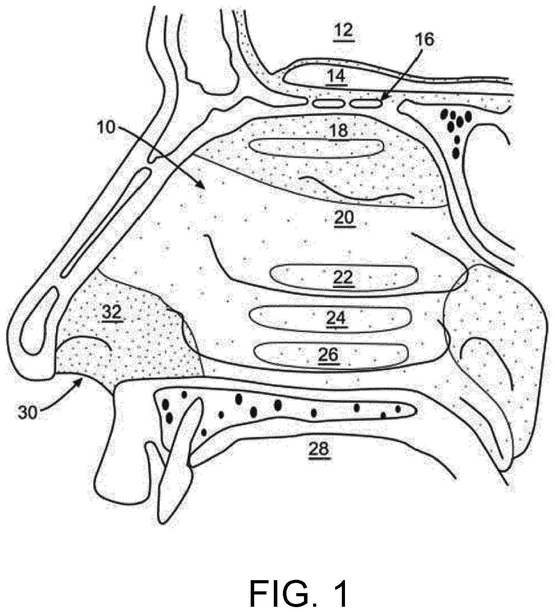

FIG. 1 is a cross sectional view, not to scale, of anatomy of a nasal cavity 10 of a person. A portion of the brain 12 is shown above the nasal cavity 10. An olfactory bulb 14 is disposed between the brain 12 and a cribriform plate 16. An olfactory mucosa 18 is below the cribriform plate 16. The nasal cavity also includes a superior turbinate 20, a middle turbinate 22, respiratory mucosa 24 and an inferior turbinate 26. Item 28 represents the palate. Injection of a pharmaceutical drug mist enters the nasal cavity 10 through the nostrils 30 and squamous mucosa 32. In order to achieve proper delivery of drugs to the blood stream using a nasal spray device, the drugs must be delivered to the respiratory mucosa 24 which is highly vascularized. Two areas that are highly vascularized are the olfactory region and the respiratory region. The respiratory region contains turbinates which increase the surface area available for drug absorption.

A common metered dose inhaler has several deficiencies that impact the quality of drug delivery to users. Many of these deficiencies are due to the difficulty some users have to coordinate inhalation with the actuation of the device to dispense the proper amount of drug from the inhaler. For some users, a spacer or valved holding chamber can be used in conjunction with the metered dose inhaler, which allows the user to dispense into the spacer and breath more naturally to inhale the drug. However, the effectiveness of these devices is still subject to proper use by the user, and creates inefficiencies and/or losses in the amount of drug delivered to the user.

A more effective method for delivering the drug dose to a user would be to adjust the amount of medicament being dispensed based on the user's behavior. For example, when the user's inhale force is greater, the flow rate of medicament may increase to deliver more fluid. On the other hand, when the user's inhale force is lighter, the flow rate of medicament may decrease to deliver less fluid. Despite the availability of a variety of devices for delivering a fluid or fluid mist to a user, there remains a need for a fluid jet ejection device that can be synchronized with the user's natural breathing cycle for more effective fluid delivery to the user.

In view of the foregoing embodiments of the disclosure provide a fluid jet ejection device and a method for controlling the delivering of a fluid to a user. The fluid jet ejection device includes a cartridge body; a fluid outlet nozzle attached to the cartridge body; a fluid jet ejection cartridge disposed in the cartridge body, the fluid jet ejection cartridge containing a fluid and an ejection head attached to the fluid jet ejection cartridge; wherein the ejection head contains a plurality of fluid ejectors thereon and a nozzle plate having a plurality of fluid ejection nozzles therein associated with the plurality of fluid ejectors configured to deliver the fluid to a user at a predetermined rate. A control system is disposed in the fluid jet ejection device that includes a differential pressure sensor for sensing an inhalation differential pressure. The control system is configured to terminate fluid delivery below a threshold inhalation differential pressure.

In another embodiment there is provided a method for controlling the delivery of a fluid to a user. The method includes providing a fluid jet ejection device containing a fluid and a fluid jet ejection head for delivering the fluid to the user. The fluid jet ejection device includes a differential pressure sensor for detecting an inhalation differential pressure during use of the fluid jet ejection device. The fluid jet ejection head is activated to deliver the fluid to the user at a predetermined rate when the differential pressure sensor senses a threshold inhalation differential pressure and activation of the fluid jet ejection head is terminated when a prescribed dosage of fluid has been delivered to the user.

In some embodiments, the control system further includes a dosage counter and the control system is further configured to terminate fluid delivery when a prescribed dosage of fluid is reached. In some embodiments, the dosage counter is configured to alert the user when the prescribed dosage of fluid is reached.

In some embodiments, the control system is further configured to initiate fluid delivery above a threshold inhalation differential pressure.

In some embodiments, the predetermined rate of fluid delivery is a constant rate of fluid delivery. In other embodiments, the predetermined rate of fluid delivery is a rate corresponding to an inhalation rate of the user.

In some embodiments, the fluid is delivered to the user as a mist.

An advantage of the device and method described herein is that a user using the device may receive the prescribed dosage of fluid while breathing normally rather than a user attempting to time inhalation with activation of a device to deliver the prescribed dosage of fluid. Another advantage of the disclosed embodiments is that the device is configured to adapt to different users using a simplified control mechanism to sense a user's normal breathing function.

### BRIEF DESCRIPTION OF THE DRAWINGS

FIG. 1 is a cross-sectional representation, not to scale, of a portion of a nasal cavity and scull of a person.
FIG. 2 is a cross-sectional view, not to scale of a fluid jet ejection device according to an embodiment of the disclosure.
FIG. 3 is a schematic cross-sectiional view, not to scale, of a portion of a an ejection head for use with the fluid jet ejection device of FIG. 2.
FIG. 4 is a plan view, not to scale, of the ejection head of FIG. 3.
FIG. 5 is a schematic illustration of a control scheme for the fluid jet ejection device of FIG. 2.
FIG. 6 is a schematic illustration of steps for activating a fluid ejector for the ejection head of FIG. 3.
FIGs. 7 is a diagramtic representation of a processor for the fluid jet ejection device of FIG. 2.
FIG. 8 is a block flow diagram for a fluid dispensing process according to a first embodiment of the disclosure.
FIG. 9 is a block flow diagram for a fluid dispensing process according to a second embodiment of the disclosure.

### DETAILED DESCRIPTION

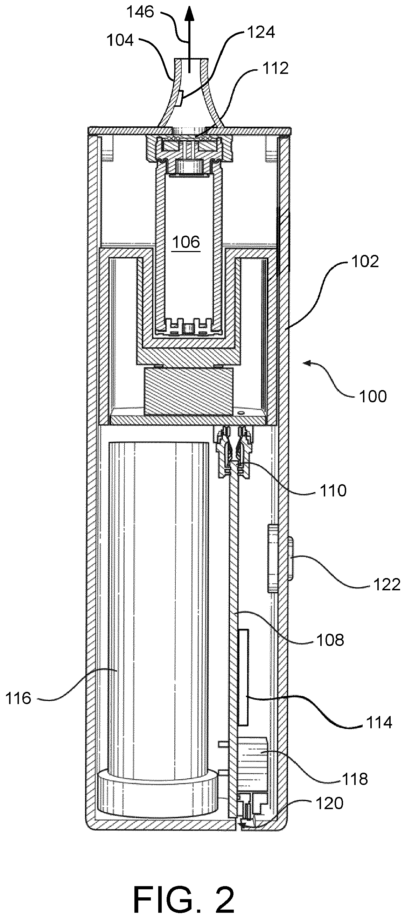

For the purposes of this disclosure, the fluid may be a mist of fluid droplets or a fluid vapor. An illustration of fluid jet ejection device 100 is shown in a cross-sectional view, not to scale, in FIG. 2. The device includes a cartridge body 102, having a fluid outlet nozzle 104 attached to the cartridge body 102. A fluid jet ejection cartridge 106 is disposed in the cartridge body 102. The fluid jet ejection cartridge 106 contains a fluid to be dispensed by the fluid jet ejection device 100. A logic board 108 is disposed in cartridge body 102 and is electrically connected via a logic board connector 110 to an ejection head 112 on the fluid jet ejection cartridge 106. As described in more detail below, the ejection head 112 includes plurality of fluid ejection nozzles and associated fluid ejectors. A processor 114 is disposed on the logic board 108 or on the ejection head 112 for executing a control algorithm to control the ejection head 112 to expel fluid from the nozzle 104 by controlling activation of fluid ejectors on the ejection head 112. The cartridge body 102 may include a rechargeable battery 116 electrically connected to the logic board 108 for providing power to the ejection head 112. A power switch 118 is used to activate the fluid jet ejection device 100. A USB input 120 may also be included to reprogram the processor 114 for use with different fluids. An activation button 122 may be used to initiate the processor 114 to deliver fluid from the fluid jet ejection device at a predetermined rate. A differential pressure sensor 124 may be provided in the nozzle 104 and electrically connected to the processor 114 to sense a differential pressure in the nozzle 104 relative to an ambient atmospheric pressure when the user is inhaling or exhaling.

A wide variety of ejection heads 112 may be used with the fluid jet ejection device 100 described above. Accordingly, the ejection head 112 may be selected from a thermal jet ejection head, a bubble jet ejection head, or a piezoelectric jet ejection head. Each of the foregoing ejection heads can produce a spray or mist of fluid when activated by control logic in the processor 114.

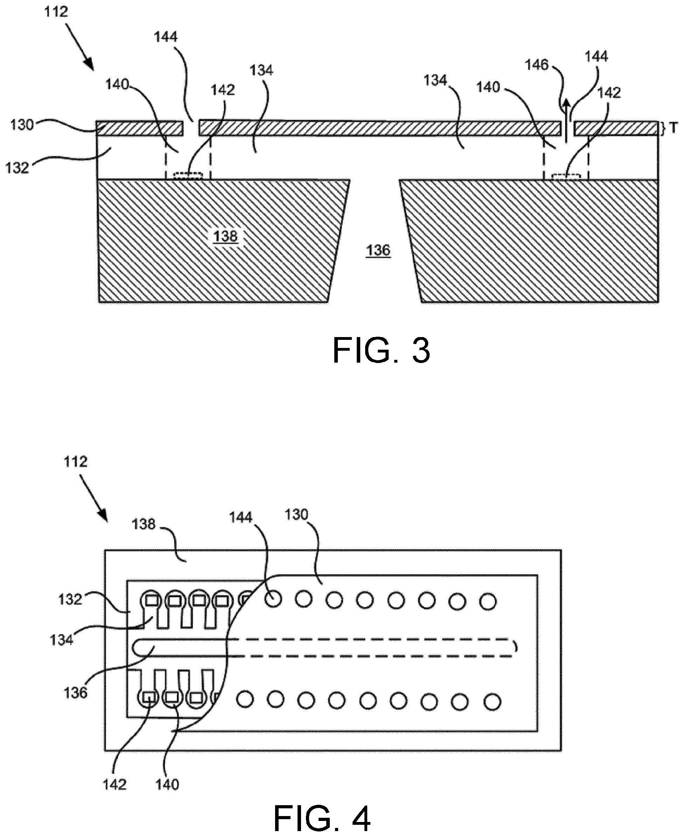

With reference to FIG. 3, there is illustrated a schematic cross-sectional view of the ejection head 112 having a nozzle plate 130 with a thickness T attached to a flow feature layer 132. The flow feature layer 132 includes a fluid flow channel 134 for directing fluid from a fluid supply via 136 in a semiconductor substrate 138 to a fluid chamber 140. The fluid chamber 140 includes a fluid ejector 142 for ejecting fluid through a nozzle hole 144 in the nozzle plate 130. A flow path through the nozzle hole 144 is indicated by arrow 146.

A plan view of the ejection head is illustrated in FIG. 4 illustrating a plurality of nozzle holes 144 disposed on both sides of the fluid supply via 136. The fluid ejectors can be activated simultaneously, sequentially, alternately, or in pre-determined groups to provide fluid droplets for fluid delivery or mist formation for delivery to the user.

When the fluid jet ejection device 100 is used as a nasal spray device, the mist of fluid droplets preferably land in the mucosa area of the nasal cavity of the user. However, the disclosure also contemplates a fluid jet ejection device that can be used for oral delivery of fluids through the mouth of a user as the user inhales. Accordingly, the embodiments of the disclosure apply to all types of inhalation devices whether oral or nasal. The fluids delivered by the device may be therapeutic fluids or any other fluids for other purposes.

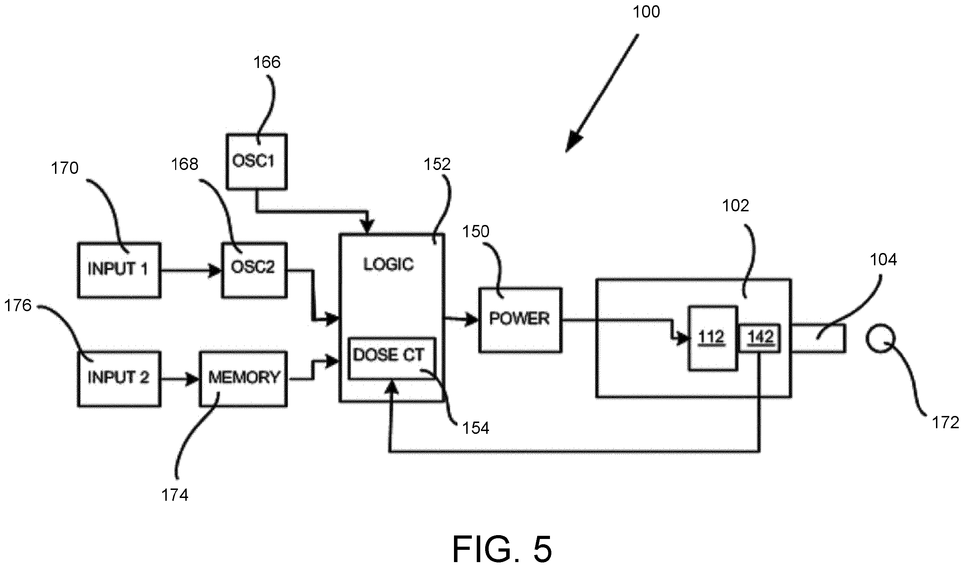

Control schemes for the fluid jet ejection device 100 are illustrated schematically in FIGs. 5-7. Referring to FIG. 5, a power circuit 150 is in electric communication with the firing mechanism, such as the fluid ejectors 142 on the ejection head 112 and causes the fluid ejectors 142 to be activated and deactivated in response to a control signal. The control signal is provided to the power circuit 150 by a logic circuit 152, which is in electric communication with the power circuit 150. The logic circuit 152 includes a dosage counter 154 which may be programmed to shut down the power circuit 150 once a predetermined dose amount of fluid has been delivered to the user.

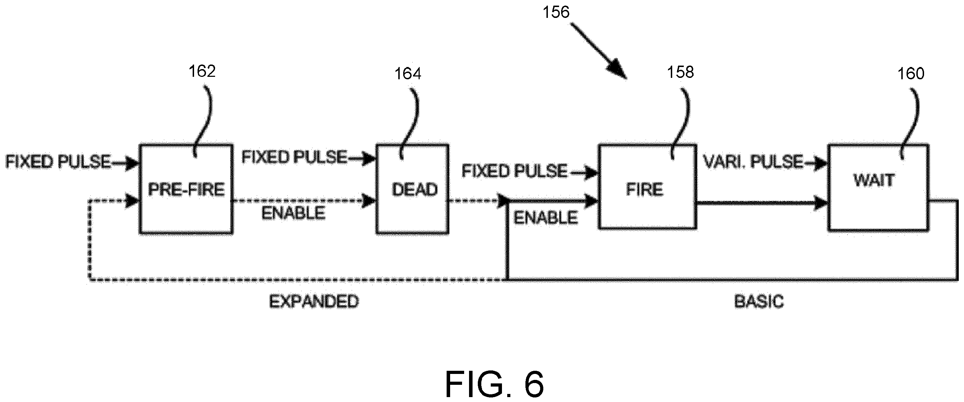

With reference to FIG. 6, a firing sequence 156 for each fluid ejector 142 on the ejection head 112 is illustrated. A basic firing sequence includes a firing step 158 where the fluid ejector 142 is activated and fluid is ejected from the fluid chamber 140. After the firing step 158, the next step in the sequence 156 is a wait step 160, where the fluid ejector 142 is deactivated and does nothing. Following the wait step 160, the firing sequence 156 is complete and may then be repeated. The flow rate of the fluid from the ejection head 112 is related to the number of firing steps 158 (i.e., fluid ejections) that occur within a given amount of time. The length of the wait step 160 determines how often firing steps 158 occur. As the wait step 160 is shortened, firing steps 158 occur more often, which results in a higher flow rate of fluid from the ejection head 112. As the wait step 160 is lengthened, firing steps 158 occur less often, which results in a lower flow rate from the ejection head 112.

The firing sequence 156 may be expanded to include a pre-fire step 162 and a dead step 164, which, in combination, allow the fluid to be preheated prior to the firing step 158. During the pre-fire step 162, the fluid ejector 142 is activated in such a way that fluid is not ejected from fluid chamber 140. For example, the fluid ejector 142 may be activated for only a very short time and then deactivated. During the dead step 164 that follows, the fluid ejector 142 remains deactivated. The dead step 164 is a time delay that allows the heat from the fluid ejector 142 to heat the fluid prior to the firing step 158. Preheating the fluid in this manner is not required, but may make the firing step 158 more accurate and predictable than if the fluid is not preheated.

The control signal determines the timing of each step in the firing sequence, including when each step occurs and how long each step lasts. The control signal is dependent on a predetermined fixed frequency signal, and it controls when the pre-fire step, the dead step, and the firing step occur and how long each step lasts. The control signal may also be dependent on a variable frequency signal, and it controls when the wait step occurs and how long it lasts. The fixed and variable frequency signals are discussed in greater detail below. In one embodiment, the power circuit is a relay, such as a power FET (Field Effect Transistor) connected between a power source and the fluid ejector 142. The control signal is an on or off signal that turns the relay on or off and thereby powers the fluid ejector 142, or un-powers the fluid ejector 142.

A predetermined fixed frequency signal and a variable frequency signal are provided to the logic circuit 152 by a first oscillator 166 and a second oscillator 168, respectively. The predetermined fixed frequency signal generated by the first oscillator 166 is a series of timing pulses, where each timing pulse is separated by a fixed time interval that remains constant over time. The pre-fire, dead, and fire steps are dependent on the control signal, which, in turn, depends on the fixed frequency signal. For that reason, the timing and length of those sequence steps remain constant from one firing sequence to the next. Thus, those steps may be very closely controlled and to remain consistent over time. In the case of a fluid jet ejection device100 for delivering respiratory drugs, for example, this type of controlled, consistent behavior ensures that a predetermined amount of medicine is delivered during each firing step

On the other hand, the variable frequency signal generated by the second oscillator 168 is a series of timing pulses, where each timing pulse is separated by a variable time interval that may vary over time. In other words, the second oscillator 168 produces pulses at a faster or slower pace depending on the length of the time interval separating timing pulses. The wait step is dependent on the variable frequency signal. For that reason, the timing and length of the wait step may vary over time.

The time interval between the timing pulses of the second oscillator 168 are varied based on a variable input signal. For example, in certain embodiments, the second oscillator 168 may comprise a voltage-controlled oscillator (VCO), which generates a VCO signal, where the frequency of that VCO signal changes based on an input. The fluid ejection device 100 includes a first input 170 for providing a variable input signal to the second oscillator 168 to vary the frequency of the VCO signal. The first input 170 may include, for example, a sensor, such as a differential pressure sensor, a dial, or other similar types of input devices. The second oscillator 168 varies the frequency of the VCO signal. The frequency of the VCO signal may be varied according to the input signal from the first input 170. In the case of a proportionate relationship, increasing the input signal results in increasing the dosage rate of fluid to the user.

The logic circuit 152 is configured (such as by programming) to vary the control signal according to the frequency of the variable frequency signal from the second oscillator 168, which causes an increase or decrease in the amount of fluid delivered to the user. Thus, the present disclosure provides a fluid jet ejection device100 wherein the flow rate of the fluid 172 from the fluid jet ejection device 100 may be determined based on a single variable input. In one embodiment the logic circuit 152 may be fixed logic that is programmed in the sense that the original design of the logic circuit is its program. Such fixed logic may not be reprogrammed without physically changing the circuit. The advantage of fixed logic is simplicity, low cost, stability, reliability and resistance to tampering. In many applications this low-cost embodiment is desirable. In other applications, more flexibility may be needed which may require reprogrammable logic. Accordingly, the USB input 120 may be used to reprogram the logic circuit 152.

In some embodiments, the fluid jet ejection device100 also includes a memory 174 for storing a control variable for setting the amount of fluid to be dispensed in each firing steps or the total amount of fluid to be dispensed to the user. In some embodiments, the amount of fluid is predetermined and is fixed into the logic circuit 152 so that a rate of fluid 172 dispensed by the fluid jet ejection device 100 is constant. In other embodiments, the amount of fluid dispensed may be changed by entering a new control variable into the memory 174 through a second input 176. The logic circuit 152, first oscillator 166, second oscillator 168, and memory 174 may be combined as a single system such as a programmable logic controller or as fully programmable processor. In some embodiments, a first input 170 may be used to vary the fluid delivery rate based on a sensed rate of inhalation of the user.

Control variables may be input via the second input 176, such as a keyboard, dial, etc. A separate variable may be assigned to each step in the firing sequence, and that control variable will determine how long that step will last. In particular, each step in the sequence is sustained until the fixed frequency signal or the variable frequency signal, depending on the sequence step involved, is equal to the control variable assigned to that particular step. Once the specified amount of time has elapsed (determined by counting pulses), the logic circuit 152 sends a control signal to the power circuit 150 to initiate the next step in sequence. As a brief example, the firing step is controlled by the control signal, which, in turn, is based on a predetermined fixed frequency signal generated by the first oscillator 166. If a control variable of 500 timing pulses, is assigned to the firing step, the firing step will be sustained until the fixed frequency signal indicates that 500 timing pulses have elapsed. At that point, the logic circuit 152 will send a control signal to the power circuit 150 to end the firing step and to begin the next step in the sequence. As mentioned above, each step in the firing sequence may be assigned a different control variable. This enables the firing sequence to be highly customizable while, at the same time, minimizing the number of inputs required to do so. The wait step is controlled by a variable frequency signal. If a control variable of 1000 is assigned to the wait step, the logic circuit 152 will sustain the wait step until 1000 pulses of the VCO signal have been counted. Since the VCO signal is variable in frequency, the length of time of the wait step is likewise variable. After the wait step is finished, when the logic has counted 1000 pulses of the VCO signal, the logic circuit 152 begins the firing sequence again.

The dosage counter 154, may be programmed to shut down the operation of the fluid jet ejection device 100 once a predetermined dose amount of fluid has been ejected. As with the control variables discussed above, the desired dosage amount may be saved to the memory 174. Each fluid ejector 142 is configured to send a signal to the dosage counter 154 at the completion of a firing sequence. In the fixed fluid rate delivery embodiment, the volume of fluid ejected during each firing sequence remains constant. Accordingly, the volume of fluid ejected may be calculated simply by knowing the number of timing sequences that have occurred. In this regard, the dosage counter 154 registers the signal from the fluid ejector 142 as a dose amount which is added to a total administered dose amount, which is stored in the memory 174. The logic circuit 152 reads the total administered dosage amount from the memory 174 and is programmed to provide a termination signal to the power circuit 150 once the administered dose amount is equal to the preprogrammed desired dosage amount. In response to the termination signal, the power circuit 150 deactivates the fluid ejectors 142 and discontinues firing sequences and, in some embodiments activates an alarm to notify the user that the required dosage has been delivered.

In another embodiment, the rate of fluid delivery by the ejection head 112 is variable and is determined by a sensed rate of inhalation of the user. In this embodiment, the differential pressure sensor 124 sends a variable signal to the second oscillator 168 which in turn varies the firing sequence logic signal to the ejection head 112 to increase or decrease the rate of fluid ejection based on the timing sequence discussed above with reference to FIG. 6.

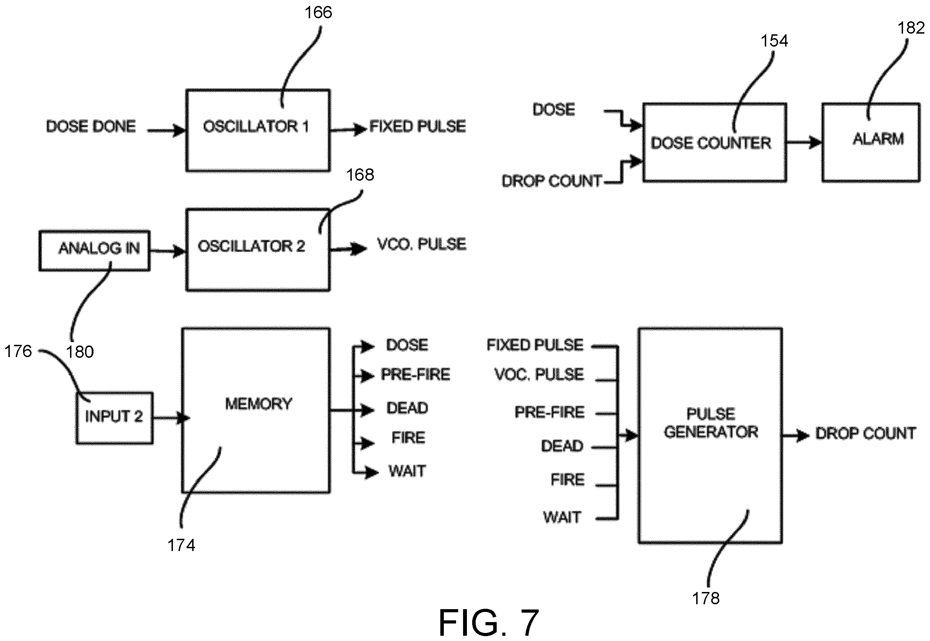

A somewhat diagrammatic representation of the programming and certain aspects of the disclosed embodiments is illustrated in FIG. 7. The memory 174 stores data and variables used by the fluid jet ejection device 100, including the desired dose rate and the total administered dose as well as the control variables for the pre-fire, dead, fire, and wait steps. That data is read by a pulse generator 178 to set the timing for each of the firing sequence steps. The pulse generator 178 may be understood to operate in a similar fashion to the power circuit 150 and the logic circuit 152 described above. The first oscillator 166 generates a fixed frequency timing pulse which is read by the pulse generator 178 to control the timing of the pre-fire, dead, and fire steps. The second oscillator 168, generates a variable frequency timing pulse which is read by the pulse generator 178 to control the timing of the wait step.

An analog input 180 such as the differential pressure sensor 124 may provide an input signal to the second oscillator 168 for varying the variable frequency timing pulse. Once a firing sequence is complete, a drop count signal is sent to the dosage counter 154, indicating that a set amount of fluid 172 has been ejected. The drop counts are saved to the memory 174. The pulse generator 178 will cause the firing sequence to repeat and the dosage counter 154 continues totaling the amount of fluid ejected, based on the number of firing sequences or drop counts, until the amount of fluid ejected is equal to the desired dosage amount. Once the pulse generator 178 reads that those amounts are equal, it stops repeating the firing sequence and the fluid jet ejection device 100 is shut down. In the first embodiment, a fixed amount of fluid is ejected during each ejection cycle as determined by a second input 176 to the memory 174. In a second embodiment, the rate of fluid ejection is determined by the sensed rate of inhalation as described above.

In operation, the fixed frequency signal, provided by the first oscillator 166, functions essentially as a clock that provides pulses at a constant interval. Each of the time-critical steps are initiated in sequential order and last for a certain amount of time or until a certain number of fixed frequency time (T*_{f}*) pulses, given by the predetermined fixed frequency signal from the first oscillator 166, have elapsed. The wait step occurs in sequential order and lasts until a certain number of variable frequency time (T*_{ν}*) pulses, given by the variable frequency signal from the second oscillator 168, have elapsed. For example, a logic circuit 152 may be configured (programmed) so that the pre-fire step, dead step, and fire steps last for T*_{f}*=5, 3, and 1 fixed frequency pulses, respectively, where each timing pulse equals 1 second. The logic circuit 152 may also be programmed so that the wait step lasts for T*_{ν}*=2 pulses. In that case, the logic circuit 152 would send a control signal (a power on signal) to the power circuit 150 to initiate the pre-fire step as soon as the first oscillator 166 is activated. That control signal turns on the power circuit 150 to initiate the pre-fire step and activate the fluid ejectors 142 for 5 time pulses. After 5 time pulses, the logic circuit 152 would send another control signal (a power off signal) that causes the power circuit 150 to deactivate the fluid ejectors 142, thus ending the pre-fire step and initiating the dead step. After 3 additional time pulses, the logic circuit 152 would send another control signal (a power on signal) that causes the power circuit 150 to re-activate the fluid ejectors 142 for a single time pulse, thus ending the dead step and activating the fire step. After 1 fixed frequency pulse, the logic circuit 152 would send a control signal (power oft) instructing the fluid ejectors 142 to deactivate for 2 variable frequency time pulses, thus ending the firing step and initiating the wait step. The actual amount of time taken for 2 variable frequency time pulses will vary, based on the input signal from the analog input 180. For example, at a first input from the analog input 180, each timing pulse may require 1 second. However, at a second input from the analog input, each timing pulse may require 5 seconds. After 2 variable frequency time pulses, the entire firing sequence would repeat.

Referring back to FIG. 2, when a user inhales, airflow in the direction indicated by the arrow 146 is caused to flow through the outlet nozzle 104. The differential pressure sensor 124 is provided near the outlet of the nozzle 104. As a user inhales, the restricted airflow within the nozzle 104 creates a low-pressure zone in the vicinity of the differential pressure sensor 124. The differential pressure sensor 124 is designed to detect the difference between the ambient pressure on the outside of the nozzle 104 and the pressure on the inside of the nozzle 104. As the strength of an inhale increases, the vacuum in the nozzle 104 increases and therefore the pressure at sensor 124 decreases compared to ambient pressure. Accordingly, the difference between ambient pressure and the pressure within the nozzle 104 increases as the user inhales. The differential pressure sensor 124 produces an analog output, preferably a voltage signal. This voltage signal is applied to the logic circuit 152 through second oscillator 168. When a predetermined threshold pressure difference is detected by the differential pressure sensor 124, the fluid ejectors 142 are activated to dispense fluid 172 to the user at a predetermined rate according to the rate stored in the memory 174. As the user continues to inhale, fluid continues to be ejected by the fluid ejectors 142. Once the pressure differential falls below the predetermined threshold pressure difference, fluid dispensing is terminated. As the user continues to breathe in an out, the logic circuit 152 continues to process the pressure differential and dispenses fluid at the predetermined rate only when the user is inhaling. Once the dosage count reaches the preselected total dosage, the dosage counter 154 activates an alarm 182 to notify the user that the treatment cycle has ended.

The frequency of second oscillator 168 varies with respect to the voltage produced by differential pressure sensor 124. When the sensor 124 detects atmospheric pressure or near atmospheric pressure, it produces a zero voltage output. The second oscillator 168 responds to the zero voltage output by producing a zero frequency output or a very low frequency output. Thus, when the user is not inhaling, no fluid is dispensed from the fluid jet ejection device 100.

When the user inhales and the sensor 124 detects a pressure below atmospheric pressure, it will produce a higher voltage signal. The voltage of the output signal produced by second oscillator 168 is proportional to the negative pressure detected by the sensor 124. In other words, as the air pressure within the outlet nozzle 104 decreases, the voltage produced by the sensor 124 increases. In response to the increasing voltage of the output from sensor 124, there is a greater flow of fluid from the fluid jet ejection device 100 to the user because the wait time decreases as the frequency of the second oscillator 168 increases. When the user inhales gently, the difference between ambient atmospheric pressure and the pressure within the nozzle 104 will be less than the pressure difference experienced during normal inhaling, and therefore the output voltage of the sensor 124 will be less, the frequency of the second oscillator 168 will be less, and the amount of fluid flow from the fluid jet ejection device 100 will be less.

According to the first embodiment, a threshold pressure differential is required for fluid delivery, indicating that the user is inhaling or breathing normally. When a threshold pressure differential below ambient atmospheric pressure is reached, the second oscillator 168 produces a voltage that is sufficient to activate the fluid ejectors 142 and thereby deliver fluid to the user at a predetermined rate according to the first embodiment of the disclosure. As the user exhales, the pressure increases to atmospheric pressure or above atmospheric pressure thereby exceeding the threshold pressure differential and terminating fluid delivery from the fluid jet ejection device 100.

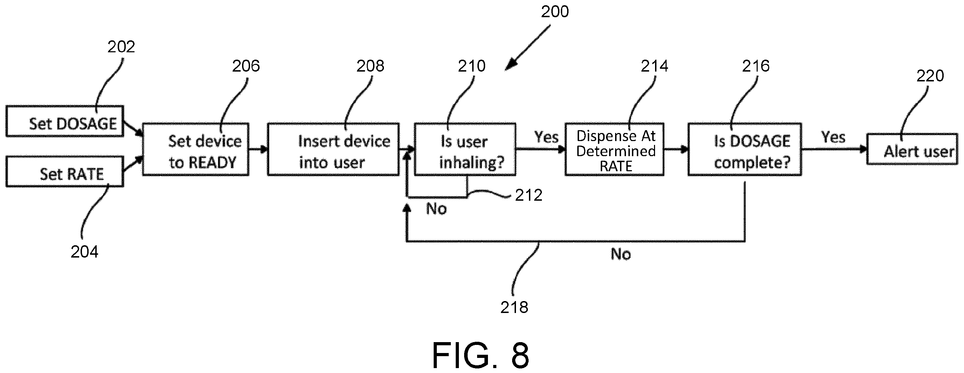

A simplified block flow diagram of the process 200 according to the first embodiment is illustrated in FIG. 8. A total dosage amount is provided as an input in step 202 and a predetermined dosage rate is provided as in input in step 204. The fluid jet ejection device 100 is activated in step 206 by pushing the power switch 118 or activated by phone or other remote device. The fluid jet ejection device 100 is then inserted into the user's mouth or nose in step 208 and the differential pressure sensor 124 determines if the user is inhaling in step 210 and if a threshold inhalation rate is sensed and is within a minimum and maximum inhalation rate. If not, as indicated by arrow 212, no fluid dispensing takes place. However, if the sensed rate of inhalation is more than or equal to the minimum threshold rate and does not exceed a maximum inhalation rate, fluid dispensing takes place according to step 214 at the predetermined fixed rate and the dosage counter 154 is indexed according to the amount of fluid dispensed. The dispensed dosage is compared to the total dosage amount in step 216. If the total dosage to be delivered to the user is not complete, step 210 is repeated as indicated by arrow 218. However, if the dosage counter 154 indicates that the total dosage has been delivered to the user, the user is alerted in step 220 that the total dosage has been delivered and the process is terminated.

In the second embodiment, the fluid delivery rate is determined by a sensed rate of inhalation by the user. Thus, fluid jet ejection device 100 may be configured to increase the flow rate continuously up to the maximum output of the ejection head 112 which would occur when the frequency of the second oscillator 168 signal equals the maximum output. Alternatively, additional logic may be incorporated into the device 100 so that there is a hard limit or cut off to the fluid flow rate that can be delivered by the ejection head 112. However, it is preferred to use an analog control system, meaning that the output of the fluid jet ejection device 100 will remain proportional to the output voltage of the sensor 124. In such case, the maximum flow rate of the fluid jet ejection device 100 will be determined by the maximum output voltage of the sensor 124. Again, there is a threshold negative pressure differential that is required to activate fluid delivery. Once the sensed inhalation terminates thereby increasing the pressure differential to ambient atmospheric pressure or above, fluid dispensing terminates. As the user continues to breath in and out, and the fluid jet ejection device 100 continues to monitor the pressure differential and dispenses fluid at a rate that is corelated to the inhalation rate of the user. As with the above, once the total amount of the prescribed dosage has been dispensed, dispensing is discontinued, and the user is then alerted that the dosage has been fully administered.

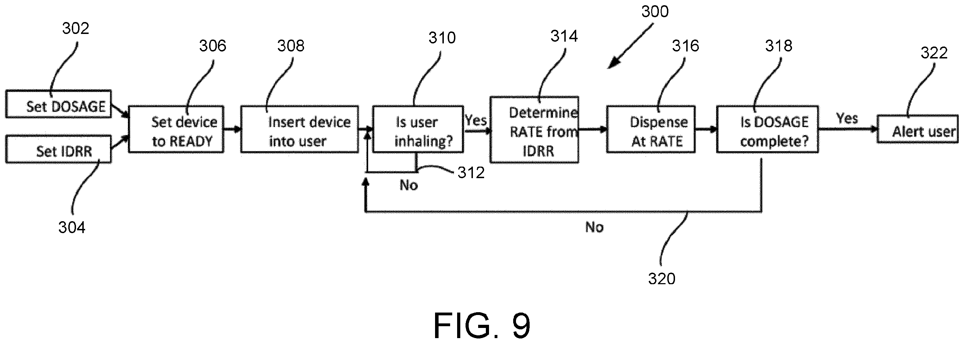

A simplified block flow diagram of the process 300 according to the second embodiment is illustrated in FIG. 9. A total dosage amount is provided as an input in step 302 and a dosage rate based on the inhalation rate, or Inhalation-Dispense-Rate-Relationship (IDRR), of the user is provided as in input in step 304. The fluid dispense device is activated in step 306 by pushing the power switch 118 or activated by phone or other remote device. The device 100 is then inserted into the user's mouth or nose in step 308 and the differential pressure sensor 124 determines if the user is inhaling in step 310 and if a threshold inhalation rate is sensed and is within a minimum and maximum inhalation rate. If not, as indicated by arrow 312, no fluid dispensing takes place. However, if the sensed rate of inhalation is within the minimum threshold rate and does not exceed a maximum inhalation rate, the dispense rate is set in step 314 based on the user's inhalation rate and fluid dispensing takes place according to step 316 at the rate set in step 314 using the predefined IDRR set in step 304. The dosage counter 154 is indexed according to the amount of fluid dispensed. The dispensed dosage is compared to the total dosage amount in step 318. If the total dosage to be delivered to the user is not complete, step 310 is repeated as indicated by arrow 320. However, if the dosage counter 154 indicates that the total dosage has been delivered to the user, the user is alerted in step 322 that the total dosage has been delivered and the process is terminated.

It is noted that, as used in this specification and the appended claims, the singular forms "a," "an," and "the," include plural referents unless expressly and unequivocally limited to one referent. As used herein, the term "include" and its grammatical variants are intended to be non-limiting, such that recitation of items in a list is not to the exclusion of other like items that can be substituted or added to the listed items.

For the purposes of this specification and appended claims, unless otherwise indicated, all numbers expressing quantities, percentages or proportions, and other numerical values used in the specification and claims, are to be understood as being modified in all instances by the term "about." Accordingly, unless indicated to the contrary, the numerical parameters set forth in the following specification and attached claims are approximations that can vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques.

### Reference Signs List

10...nasal cavity
12...brain
14...olfactory bulb
16...cribriform plate
20...superior turbinate
22...middle turbinate
24...respiratory mucosa
26...inferior turbinate
28...Item
30...nostrils
32...squamous mucosa
100...fluid delivery device
102...cartridge body
104...fluid outlet nozzle
106...fluidjet ejection cartridge
108...logic board
110...logic board connector
112...ejection head
114...processor
116...rechargeable battery
118...power switch
120...USB input
122...activation button
124...differential pressure sensor
130...nozzle plate
132...flow feature layer
134...fluid flow channel
136...fluid supply via
138...semiconductor substrate
140...fluid chamber
142...fluid ejector
144...nozzle hole
146, 212, 218, 312, 320...arrow
150...power circuit
152...logic circuit
154...dosage counter
156...firing sequence
158...firing step
160...wait step
162...pre-fire step
164...dead step
166...first oscillator
168...second oscillator
170...first input
172...fluid
174...memory
176...econd input
178...pulse generator
180...analog input
182...alarm
200, 300...process
202, 204, 206, 208, 210, 214, 216, 302, 304, 306, 308, 310, 314, 316, 318, 322...step
FET...power
T...thickness

## Claims

1. A fluid jet ejection device (100) comprising:
a cartridge body (102);
a fluid outlet nozzle (104) attached to the cartridge body (102);
a fluid jet ejection cartridge (106) disposed in the cartridge body (102), the fluid jet ejection cartridge (106) containing a fluid and an ejection head (112) attached to the fluid jet ejection cartridge (106); wherein the ejection head (112) contains a plurality of fluid ejectors (142) thereon and a nozzle plate (130) having a plurality of fluid ejection nozzles therein associated with the plurality of fluid ejectors (142) configured to deliver the fluid to a user at a predetermined rate; and
a control system disposed in the fluid jet ejection device(100) comprising a differential pressure sensor (124) for sensing an inhalation differential pressure wherein the control system is configured to terminate fluid delivery below a threshold inhalation differential pressure.

2. The fluid jet ejection device (100) of claim 1, wherein the control system further comprises a dosage counter (154) and the control system is further configured to terminate fluid delivery when a prescribed dosage of the fluid is reached.

3. The fluid jet ejection device (100) of claim 1, wherein the control system is further configured to initiate fluid delivery above a threshold inhalation differential pressure.

4. The fluid jet ejection device (100) of claim 1, wherein the predetermined rate of fluid delivery is a constant rate of fluid delivery.

5. The fluid jet ejection device (100) of claim 1, wherein the predetermined rate of fluid delivery is a rate corresponding to an inhalation rate of the user.

6. The fluid jet ejection device (100) of claim 1, wherein the fluid is delivered to the user as a mist.

7. A method for controlling a delivery of a fluid to a user, the method comprising:
providing a fluid jet ejection device (100) containing a fluid and a fluid jet ejection head (112) for delivering the fluid to the user;
providing a differential pressure sensor (124) in the fluid jet ejection device (100) for detecting an inhalation differential pressure during use of the fluid jet ejection device (100);
activating the fluid jet ejection head (112) to deliver the fluid to the user at a predetermined rate when the differential pressure sensor (124) senses a threshold inhalation differential pressure; and
terminating the activation of the fluid jet ejection head (112) when a prescribed dosage of the fluid has been delivered to the user.

8. The method of claim 7, wherein the fluid jet ejection device (100) includes a dosage counter (154), further comprising alerting the user when the prescribed dosage of the fluid has been delivered to the user.

9. The method of claim 7, wherein the predetermined rate of fluid delivery is a constant rate of fluid delivery.

10. The method of claim 7, wherein the predetermined rate of fluid delivery is a rate corresponding to an inhalation rate of the user.

11. The method of claim 7, further comprising terminating the delivery of fluid when the differential pressure sensor (124) senses an inhalation differential pressure below the threshold inhalation differential pressure.
